(19) Europäisches Patentamt
European Patent Office
Office européen des brevets

(11) Publication number: 0 239 075 A2

# (12) EUROPEAN PATENT APPLICATION

(21) Application number: 87104321.2

(22) Date of filing: 24.03.87

(51) Int. Cl.[4]: C12N 15/00 , C12P 21/02 , C12N 1/18

The applicant has filed a statement in accordance with Rule 28 (4) EPC (issue of a sample only to an expert). Accession number(s) of the deposit(s): FERM BP - 1001; FERM BP - 612.

(30) Priority: 25.03.86 JP 66807/86

(43) Date of publication of application: 30.09.87 Bulletin 87/40

(84) Designated Contracting States: DE FR GB

(71) Applicant: KYOWA HAKKO KOGYO CO., LTD.
6-1, Ohte-Machi 1-chome
Chiyoda-ku Tokyo-to(JP)

(72) Inventor: Hosoi, Noriko
Popuraoka Coopu 16-104 2-21-2, Naruse
Machida-shi Tokyo(JP)
Inventor: Sekine, Susumu
Popuraoka Coopu 1-102 2-131, Naruse
Machida-shi Tokyo(JP)
Inventor: Itoh, Seiga
218-14, Aza Hachimannishi Aihara
Sagamihara-shi Kanagawa-ken(JP)

(74) Representative: Vossius & Partner
Siebertstrasse 4 P.O. Box 86 07 67
D-8000 München 86(DE)

## (54) Process for producing fish growth hormone polypeptide.

(57) According to the present invention, a recombinant DNA incorporated with a DNA coding for a fish growth hormone polypeptide and a yeast containing the recombinant DNA were obtained and they can be used for mass production of the fish growth hormone polypeptide.

EP 0 239 075 A2

# PROCESS FOR PRODUCING FISH GROWTH HORMONE POLYPEPTIDE

The present invention relates to a process for producing a fish growth hormone polypeptide using a microorganism containing a recombinant DNA incorporating a DNA coding for the fish growth hormone polypeptide. The fish growth hormone is expected to have various uses in the industrial field of fish cultivation.

The present inventors studied methods of producing fish growth hormones by recombinant DNA technology. As the result, the present inventors successfully recovered a DNA complementary to a fish growth hormone polypeptide and usable in the production of fish growth hormones, and produced in a recombinant DNA containing the DNA and a microorganism containing the recombinant DNA. That is, a messenger RNA (mRNA) was extracted from the salmon pituitary gland and a DNA (cDNA) complementary to the mRNA was synthesized. Then, a DNA probe corresponding to the amino acid sequence around the N-terminal of the salmon growth hormone was synthesized, and a salmon growth hormone gene was cloned by selecting a cDNA which hybridized with the DNA probe. Further, the base sequence of the cDNA was determined as disclosed in JP-A-15699/86.

Fish growth hormones have a stimulating effect for the growth of fish and are useful as a component of baits for fish cultivation. The amount of the growth hormone provided by the recovery from the fish pituitary gland is limited. Therefore, it had been desired that a process for providing a large amount of fish growth hormones at low costs is developed.

The present inventors further studied and found that a large amount of the salmon growth hormone polypeptide is formed and accumulated in a medium by culturing a microorganism belonging to Escherichia coli and containing a recombinant DNA wherein a cDNA coding for the salmon growth hormone is incorporated, as disclosed in JP-A-93197/86 and 210100/86.

However, many problems to be settled remained. For example, the protein product from Escherichia coli was sometimes contaminated with endotoxins which have to be removed by uneconomical purification steps. Further, the protein produced in a large amount in Escherichia coli was accumulated as almost inactive proteins since the biochemical circumstance in Escherichia coli is quite different from the circumstance in which the protein of a higher living to be produced can actively be present. Therefore, the development of an economical process for mass production of an active fish growth hormone which does not contain endotoxins is desired.

The present invention provides a process for producing a salmon growth hormone polypeptide in a large amount by culturing a yeast containing a recombinant DNA incorporating a DNA coding for the salmon growth hormone. According to the present invention, an active fish growth hormone which does not contain endotoxins can be provided.

Fig. 1 is a flow sheet for constructing the plasmid psGHIJL2.

Fig. 2 is a flow sheet for constructing the plasmid pYSHB5.

In the drawings, the part illustrated by a black point shows lipoprotein terminator (Tlpp).

The present invention provides a process for producing a fish growth hormone polypeptide, for example, the polypeptide having the nucleotide sequence as illustrated in Table 1 by recombinant DNA technology.

Table 1

```
         10        20        30        40        50        60
ATGGGACAAGTGTTTCTGCTGATGCCAGTCTTACTGGTCAGTTGTTTCCTGAGTCAAGGG
MetGlyGlnValPheLeuLeuMetProValLeuLeuValSerCysPheLeuSerGlnGly

         70        80        90       100       110       120
GCAGCGATAGAAAACCAACGGCTCTTCAACATCGCGGTCAGTCGGGTGCAACATCTCCAC
AlaAlaIleGluAsnGlnArgLeuPheAsnIleAlaValSerArgValGlnHisLeuHis

        130       140       150       160       170       180
CTATTGGCTCAGAAAATGTTCAATGACTTTGACGGTACCCTGTTGCCTGATGAACGCAGA
LeuLeuAlaGlnLysMetPheAsnAspPheAspGlyThrLeuLeuProAspGluArgArg

        190       200       210       220       230       240
CAGCTGAACAAGATATTCCTGCTGGACTTCTGTAACTCTGACTCCATCGTGAGCCCAGTC
GlnLeuAsnLysIlePheLeuLeuAspPheCysAsnSerAspSerIleValSerProVal

        250       260       270       280       290       300
GACAAGCACGAGACTCAGAAGAGTTCAGTCCTGAAGCTGCTCCACATTTCTTTCCGTCTG
AspLysHisGluThrGlnLysSerSerValLeuLysLeuLeuHisIleSerPheArgLeu

        310       320       330       340       350       360
ATTGAATCCTGGGAGTACCCTAGCCAGACCCTGATCATCTCCAACAGCCTAATGGTCAGA
IleGluSerTrpGluTyrProSerGlnThrLeuIleIleSerAsnSerLeuMetValArg

        370       380       390       400       410       420
AACGCCAACCAGATCTCTGAGAAGCTCAGCGACCTCAAAGTGGGCATCAACCTGCTCATC
AsnAlaAsnGlnIleSerGluLysLeuSerAspLeuLysValGlyIleAsnLeuLeuIle

        430       440       450       460       470       480
ACGGGGAGCCAGGATGGCGTACTGAGCCTGGATGACAATGACTCTCAGCAGCTGCCCCCC
ThrGlySerGlnAspGlyValLeuSerLeuAspAspAsnAspSerGlnGlnLeuProPro

        490       500       510       520       530       540
TACGGAAACTACTACCAGAACCTGGGGGGCGACGGAAACGTCAGGAGGAACTACGAGTTG
TyrGlyAsnTyrTyrGlnAsnLeuGlyGlyAspGlyAsnValArgArgAsnTyrGluLeu

        550       560       570       580       590       600
TTGGCATGCTTCAAGAAGGACATGCACAAGGTCGAGACCTACCTGACCGTCGCCAAGTGC
LeuAlaCysPheLysLysAspMetHisLysValGluThrTyrLeuThrValAlaLysCys

        610       620       630
AGGAAGTCACTGGAGGCCAACTGCACTCTGTAG
ArgLysSerLeuGluAlaAsnCysThrLeu***
```

A DNA (cDNA) complementary to an mRNA of a fish growth hormone is prepared using the mRNA as a template and then a recombinant plasmid incorporating the cDNA is prepared. The recombinant plasmid is introduced in a host yeast strain. The salmon growth hormone polypeptide can economically be produced in a large amount by culturing the yeast strain carrying the recombinant plasmid.

The DNA and recombinant plasmid of the present invention are prepared by the following general method.

Total RNA is prepared from the pituitary gland of a salmon (Oncorhynchus keta) and passed through an oligo dT cellulose column to isolate RNA having polyadenylic acid [poly(A)RNA]. A double stranded RNA is synthesized using the poly(A)RNA as a template and a reverse transcriptase. A recombinant DNA is obtained using in vitro recombinant DNA technology by inserting the synthetic DNA into a vector DNA such as Escherichia coli plasmid DNA.

A process for producing the DNA and recombinant DNA of the present invention is explained in detail below.

The pituitary gland is excised from captured salmons and immediately freezed in liquid nitrogen. Guanidinium isothiocyanate is added to the freezed pituitary gland and the pituitary gland is disrupted and solubilized. Then, the solubilized pituitary gland is put on CsCl solution layer and subjected to ultra centrifugation to obtain whole cytoplasmic RNA as a precipitate. Alternatively, LiCl is added to the solubilized matter with guanidinium isothiocyanate to recover only RNA as a precipitate.

The extracted RNA is dissolved in an NaCl or KCl hypertonic solution (for example, 0.5 M) and passed through an oligo(dT) cellulose column to allow mRNA having polyadenylic acid [poly(A)] to be adsorbed on the column. Elution is carried out with water or a hypotonic salt solution such as 10 mM Tris-HCl buffer to isolate the mRNA having poly(A).

Synthesis of cDNA and insertion of the cDNA into a vector are carried out according to the method of Okayama-Berg [Okayama & Berg; Mol. Cell. Biol. 2, 161 (1982)] as follows.

First, a vector primer is synthesized. A vector, e.g. pCDVI, is treated with KpnI in an adequate solution such as a solution consisting of Tris-HCl buffer (e.g. pH 7.5, 10 mM), $MgCl_2$ (e.g. 6 mM) and NaCl (e.g. 10 mM) to cut pCDVI at KpnI site. The DNA is incubated with terminal deoxynucleotidyltransferase at an appropriate temperature (e.g. 37°C) for an appropriate period (e.g. 20 minutes) in a solution consisting of Tris-HCl buffer (e.g. pH 6.8, 20 mM), sodium cacodylate (e.g. 140 mM), $CoCl_2$ (e.g. 1 mM), dithiothreitol (e.g. 0.1 mM) and dTTP (e.g. 0.25 mM) to add about 60 thymidyl residues to the both 3′ ends of the vector DNA. Then, the DNA is digested with EcoRI in a solution consisting of Tris-HCl buffer (e.g. pH 7.5, 10 mM), $MgCl_2$ (e.g. 6 mM) and NaCl (e.g. 100 mM). The digested solution is fractionated by low-gelling-temperature agarose gel electrophoresis (referred to as LGT method hereinafter) [Lars Wieslander: Analytical Biochemistry, 98, 305 (1979)] to recover a DNA fragment of about 3.1 Kb. Then, the DNA is dissolved in a NaCl or KCl hypertonic solution (e.g. 0.5 M) and passed through a poly(dA) cellulose column to allow only vector primer molecules having poly(T) to be adsorbed on the column. Elution is carried out with water or a hypotonic salt solution such as 10 mM Tris-HCl buffer to isolate only the vector primer molecule with poly-(T).

Then, a linker DNA is synthesized as follows. For example, pLI DNA is treated with PstI in an appropriate solution such as a solution consisting of a Tris-HCl buffer (e.g. pH 7.5, 10 mM), $MgCl_2$ (e.g. 6 mM) and NaCl (e.g. 50 mM) to cut pLI at PstI site. The DNA is treated by the same method as in the synthesis of the vector primer except that dGTP is added in place of dTTP, and about 15 oligo(dG) chains are added. The DNA is cut with HindIII in an appropriate solution such as a solution consisting of Tris-HCl buffer (e.g. pH 7.5, 10 mM), $MgCl_2$ (e.g. 6 mM) and NaCl (e.g. 60 mM). A DNA fragment of about 0.5 Kb is fractionated by agarose gel electrophoresis and recovered with DEAE paper. Thus, a linker DNA is obtained.

The thus obtained poly(A)RNA, vector primer and linker DNA are used to synthesize cDNA as follows. The poly(A)RNA and vector primer DNA are reacted with a reverse transcriptase at an appropriate temperature (e.g. 37°C) for an appropriate method (e.g. 40 minutes) in a solution consisting of Tris-HCl buffer (e.g. pH 8.3, 50 mM) $MgCl_2$ (e.g. 8 mM), KCl (e.g. 30 mM), dithiothreitol (e.g. 0.3 mM), and dATP, dTTP, dCTP and dGTP (e.g. each 2 mM). About 15 oligo(dC) chains are added at the 3′ ends of the thus obtained RNA-DNA double strand in the same conditions and operation as in the case of the addition of (dT) chains to the vector primer except that dTTP is replaced with dCTP. The DNA is cut with HindIII in a solution consisting of Tris-HCl buffer (e.g. pH 7.5, 10 mM), $MgCl_2$ (e.g. 6 mM) and NaCl (e.g. 60 mM). The previously prepared linker DNA is mixed with the DNA and the mixture is incubated with Escherichia coli DNA ligase at an appropriate temperature (e.g. 12°C) for an appropriate period (e.g 16 hours) in a solution consisting of Tris-HCl buffer (e.g. pH 7.5, 20 mM), $MgCl_2$ (e.g. 4 mM), $(NH_4)_2SO_4$ (e.g. 10 mM), KCl (e.g. 0.1 M) and β-nicotinamide adenine dinucleotide (β-NAD) (e.g. 0.1 mM) to circularize the cDNA and linker DNA. To the reaction solution are added 40 μM (final concentration) each dATP, dTTP, dGTP and dCTP. Escherichiacoli DNA ligase, Escherichia coli DNA polymerase I and Escherichia coli ribonuclease H are added to replace the RNA part with DNA and to obtain a recombinant plasmid containing a complete double stranded cDNA.

An Escherichia coli strain, e.g. Escherichia coli c600SF8 is transformed with the thus obtained recombinant plasmid, for example, by the method of Scott, et al. [Katsuya Shigesada: Saibo Kogaku (Cell Engineering), 2, 616 (1983)]. Since an ampicillin resistance gene exists in the recombinant plasmid mentioned above, the Escherichia coli transformants are resistant to ampicillin.

Selection of a microorganism strain carrying a new recombinant plasmid DNA having a gene complementary to the mRNA of fish growth hormone from the ampicillin-resistant ($Ap^R$) strains is carried out as follows. That is, the transformants obtained above are fixed on a nitrocellulose filter and a synthetic DNA probe having a DNA sequence which is presumed from the amino acid sequence of a known salmon growth hormone polypeptide is hybridized thereto to select the transformant showing strong hybridization [the Method of Grunstein-Hogness, Proc. Natl. Acad. Sci., USA, 72, 3961 (1975)]. The probe DNA is synthesized by a conventional triester method [J. Am. Chem. Soc., 97, 7327 (1975)]. Selection by the synthesized DNA probe is more definitely carried out by the method of Southern, et al. [J. Mol. Biol., 98, 503 (1975)] and a recombinant plasmid DNA having the gene complementary to a salmon growth hormone mRNA is identified by the same method mentioned above.

psGH1 (JP-A-15699/86) is an example of the thus obtained recombinant plasmids. The plasmid can be used as a source of the DNA coding for salmon growth hormone.

Production of salmon growth hormone polypeptide by the expression of the DNA coding for salmon growth hormone in a yeast:

The DNA coding for salmon growth hormone is cut out from the plasmid carrying the DNA and inserted into a vector DNA. The thus obtained recombinant DNA is incorporated in a yeast and the thus obtained transformant is cultured to accumulate salmon growth hormone polypeptide in cultured cells. Then salmon growth hormone is recovered from the cells.

As the plasmid containing the DNA coding for salmon growth hormone, psGH1 mentioned above, psGH14 described in JP-A-210100/86 and psGHIB2 described in JP-A-93197/86 are preferable examples.

psGHIB2 used in Example can be obtained as follows.

MboII-Sal I digestion fragment coding for salmon growth hormone and Sal I-BamHI digestion fragment are separately isolated from psGHI and HindIII-BamHI digestion fragment containing tryptophan promoter from pGEL1 is isolated. On the other hand, the synthetic DNA linker as set forth below is prepared.

```
HindIII                                        MboII
5' A G C T T A T G A T A G A A A A C 3'
        3' A T A C T A T C T T T T 5'
               Met   Ile   Glu   Asn
                      1     2     3
```

The DNA fragments and synthetic DNA linker described above are ligated with T4 DNA ligase to obtain the recombinant plasmid psGHIB2.

As the vector DNA, any vector can be used so long as the DNA incorporated therein can be expressed in a yeast. Vectors having markers such as replication origin 2 μm sequence of yeast, $Ap^R$ (ampicillin resistance), and leu2 (leucine non-requirement) are used so as to maintain plasmids in yeast cells and make it possible to select transformants in both Escherichia coli and yeast. Preferably, a vector DNA which has an appropriate promoter such as the promoter of a gene responsible for yeast regulatory acid phosphatase ($P_{PHO5}$) [Richard. A. Kramer, et al., Proc. Natl. Acad. Sci. USA, 81, 367 - 370 (1984)], the promoter of phosphoglyceratekinase gene ($P_{PGK}$) [M.J. Dobson, et al., Nucleic Acids Research, 10, 2625 - 2637 (1982)], the promoter of alcohol dehydrogenase gene ($P_{ADH1}$) [Grant A. Bitter, et al., Gene, 32, 263 - 274 (1984)] and the promoter of glyceraldehyde-3-phosphate dehydrogenase gene ($P_{GAP-DH}$) [Holland, et al ., J. Biol. Chem., 254, 9839 (1979)] and which can incorporate a DNA downstream from the promoter is employed.

A preferred example of the vector DNA is plasmid pAM82. pAM82 is a plasmid as illustrated in Fig. 2, and the structure thereof and process for producing it are described in Proc. Natl. Acad. Sci., USA, 80, 1 - 5 (1983).

Recombination of the DNA coding for the polypeptide and the vector DNA is carried out by a general recombinant DNA technology wherein both DNAs are digested with restriction enzymes and ligated using T4 DNA ligase.

In the case of psGHIJL2 and pAM82, as illustrated in Fig. 2, Xhol-Pstl digestion fragment coding for salmon growth hormone is obtained from psGHIJL2 and Xhol-Pst I digestion fragment containing the promoter of yeast regulatory acid phosphatase ($P_{PHO5}$) and Pstl-Pstl digestion fragment are separately obtained from pAM82.

The DNA fragments mentioned above are ligated with T4 DNA ligase to obtain a recombinant plasmid pYSHB5 illustrated in Fig. 2. The plasmid has the DNA coding for mature salmon growth hormone.

Reaction conditions required in the recombinant DNA technology described above are generally as follows.

Digestion of the DNA with restriction enzymes is usually carried out by reacting 0.1 to 20 μg of DNA with 0.1 - 100 units, preferably 1 - 3 units of restriction enzyme per 1 μg of DNA in a mixture of 2 - 200 mM, preferably 10 -40 mM Tris-HCl (pH 6.0 - 9.5, preferably pH 7.0 to 8.0), 0 -200 mM NaCl and 2 - 30 mM, preferably 5 - 10 mM $MgCl_2$ at 20 - 70°C (optimum temperature depends on restriction enzymes used) for 15 minutes to 24 hours. Reaction is usually stopped by heating at 55 - 75°C for 5 - 30 minutes, or alternatively by inactivating the restriction enzyme with a reagent such as phenol and diethylpyrocarbonate.

Purification of the DNA fragments formed by digestion with restriction enzymes is carried out by LGT method or polyacrylamide gel electrophoresis.

Ligation of the DNA fragments is carried out with 0.3 - 10 units of T4 DNA ligase in a mixture of 2 - 200 mM, preferably 10 - 40 mM Tris-HCl (pH 6.1 - 9.5, preferably 7.0 - 8.0), 2 - 20 mM, preferably 5 - 10 mM $MgCl_2$, 0.1 - 10 mM preferably 0.5 - 2.0 mM ATP and 1 - 50 mM, preferably 5 -10 mM dithiothreitol at 1 - 37°C, preferably 3 - 20°C for 15 minutes to 72 hours, preferably 2 - 20 hours.

The recombinant plasmid DNA formed by the ligation reaction is introduced into an Escherichia coli, if necessary, by the transformation method of Cohen, et al. [S.N. Cohen, et al.: Proc. Natl. Acad. Sci. USA, 69, 2110 (1972)].

Isolation of the recombinant plasmid DNA from Escherichia coli carrying the DNA is carried out by the method described in Example 1 or the method of Birnboim, et al. [H.C. Birnboim, et al.: Nucleic Acids Res. 7, 1513 (1979)].

Plasmid DNA is digested with 1 - 10 kinds of restriction endonucleases and the cleavage sites are examined by agarose gel electrophoresis or polyacrylamide gel electrophoresis. Further, if necessary, the base sequence of the DNA is determined by the method of Maxam-Gilbert [Proc. Natl. Acad. Sci. 74, 560 (1977)] or the method of Sanger [Sanger, et al., Proc. Natl. Acad. Sci. USA, 74, 5463 (1977)]; Amersham Co., M13 cloning and sequencing handbook].

A recombinant plasmid DNA can be made as mentioned above.

The recombinant DNA as prepared is, if necessary, incorporated into a yeast by the transformation method of Ito, etal. [Hisao Ito et al.: Journal of Bacteriology 153, 163 - 168 (1983)], etc.

As the yeast used for transformation, those in which a DNA coding for fish growth hormone polypeptide is expressed and the polypeptide is produced when the recombinant plasmid DNA is incorporated into a yeast and when the yeast is cultured are employed. Preferably, strains belonging to the genus Saccharomyces, for example Saccharomyces cerevisiae SHY3 is employed.

Isolation of the DNA from the yeast carrying the recombinant DNA is carried out by the method of Kim, et al. [Proc. Natl. Acad. Sci. USA, 77, 2119 - 2123 (1980)], etc.

The salmon growth hormone polypeptide of the present invention is produced by the following method.

That is, Saccharomyces cerevisiae SHY3 (ATCC44771) is transformed with a plasmid such as pYSHB5 and a yeast strain carrying pYSHB5 is selected from the leucine non-requiring colonies (having leu2 gene). The yeast strain SHY3/pYSHB5 carrying pYSHB5 is cultured in a medium free from inorganic phosphate to produce the salmon growth hormone polypeptide in the cultured cells.

As the medium, either a synthetic medium or a natural medium can be used so long as it is suitable for the growth of yeast and the production of the salmon growth hormone polypeptide.

As a carbon source, glucose, maltose, sucrose, fructose, lactose, glycerol, mannitol, sorbitol, etc. may be used.

As a nitrogen source, $NH_4Cl$, $(NH_4)_2SO_4$, yeast extract, polypeptone, casamino acid, meat extract, Bactotryptone, malt extract, corn steep liquor, etc. may be used.

In addition, nutrients such as $K_2HPO_4$, $KH_2PO_4$, NaCl, $MgSO_4$, $MgCl_2$, $FeSO_4$, $CaCl_2$ and vitamine $B_1$ may be used.

Culturing is carried out at pH 4.0 - 10.0 and at 15 - 37°C with aeration and stirring.

After culturing for 8 - 100 hours, the salmon growth hormone polypeptide is accumulated within cultured cells. The collected cells are treated with zymolyase, disrupted by supersonic waves and subjected to centrifugation. The thus obtained supernatant fluid is subjected to extraction according to a conventional method for extraction of polypeptides to recover the polypeptide.

Detection of the polypeptide is carried out by heat-dissolving the cultured cells directly in Sample buffer of Laemmli [Laemmli, Nature, 227, 680 (1970)] and by subjecting to SDS-polyacrylamide gel [the method of Laemmli: the reference mentioned above] and Coomassie Brilliant Blue staining.

JP-A-15699/86, 93197/86 and 210100/86, combined together, correspond to EP-A-0166444A2.

Certain specific embodiments of the invention are illustrated by the following representative examples.

Example 1

Construction of recombinant plasmid pYSHB5 coding for the mature salmon growth hormone:

In this example, 5 μg of plasmid pSGHIB2 [Proc. Natl. Acad. Sci., USA, 82, 4306 - 4310 (1985)] containing a DNA coding for a salmon growth hormone was dissolved in 40 μl of a solution comprising 10 mM Tris-HCl (pH 7.5), 7 mM $MgCl_2$, 6 mM 2-mercaptoethanol and 150 mM NaCl (referred to as "Y-150 buffer solution" hereinafter). Then, 10 units of restriction enzyme NsiI (product of New England Biolabs Co.) was added and digestion reaction was carried out at 37°C for 3 hours. After phenol and chloroform extraction and ethanol precipitation, the DNA fragment was dissolved in 40 μl of a buffer solution comprising 50 mM Tris-HCl (pH 7.6), 7 mM $MgCl_2$, 6 mM 2-mercaptoethanol, 0.25 mM dATP, 0.25 mM dCTP, 0.25 mM dGTP and 0.25 mM dTTP (referred to as "DNA polymerase I buffer solution" hereinafter) and 6 units of Escherichia coli DNA polymerase I•Klenow fragment (product of Takara Shuzo Co. the same is used hereinafter) was added. Reaction was carried out at 15°C for 2 hours to convert 3'-protruding ends formed by NsiI digestion to flush ends. Reaction was stopped by phenol extraction nd chloroform extraction and ethanol precipitation were carried out. The DNA fragment was dissolved in 40 μl of a buffer solution comprising 10 mM Tris-HCl (pH 7.5), 7 mM $MgCl_2$, 6 mM 2-mercaptoethanol and 60 mM NaCl (referred to as "Y-60 buffer solution" hereinafter) and 8 units of HindIII (product of Takara Shuzo Co.; the restriction enzymes as used hereinafter are all products of Takara Shuzo Co., unless otherwise specified) was added. Reaction was carried out at 37°C for 3 hours. After heat treatment at 65°C for 10 minutes, 0.5 μg of a smaller plasmid DNA fragment (832 bp) containing the gene coding for the salmon growth hormone was recovered by LGT method (this method is hereinafter used for purifying DNA fragments unless otherwise specified).

Separately, 5 μg of plasmid psGHIB2 was dissolved in 40 μl of a solution comprising 10 mM Tris-HCl (pH 7.5), 7 mM $MgCl_2$, 6 mM 2-mercaptoethanol and 100 mM NaCl (referred to as "Y-100 buffer solution" hereinafter). Ten units of restriction enzyme BamHI was added and digestion reaction was carried out at 30°C for 3 hours. Then, after phenol and chloroform extraction and ethanol precipitation, the DNA fragment was dissolved in 40 μl of DNA polymerase I buffer solution. Six units of Escherichia coli DNA polymerase I•Klenow fragment was added and reaction was carried out at 15°C for 2 hours to convert 5'-protruding ends formed by BamHI digestion to flush ends. Reaction was stopped by phenol extraction. After chloroform extraction and ethanol precipitation, the DNA fragment was dissolved in 40 μl of Y-60 buffer solution. Eight units of HindIII was added and digestion reaction was carried out at 37°C for 3 hours. After heat treatment at 65°C for 10 minutes, about 1 μg of a larger plasmid DNA (about 2.7 Kb) containing a lipoprotein-terminator (Tlpp), a Ptrp-Ptrp tandem promoter and an ampicillin resistance gene was recovered by LGT method.

About 0.1 μg of DNA fragment of 832 bp and about 0.2 μg of DNA fragment of about 2.7 Kb obtained as mentioned above from psGHIB2 were dissolved in 20 μl of a buffer solution comprising 50 mM Tris-HCl (pH 7.5), 10 mM $MgCl_2$, 10 mM dithiothreitol and 1 mM ATP (referred to as "T4 DNA ligase buffer solution" hereinafter). Two units of T4 DNA ligase (product of Takara Shuzo Co. the same is used hereinafter) was added and ligation reaction was carried out at 4°C for 18 hours.

Escherichia coli HB101 [Journal of Molecular Biology, 41, 459 - 472 (1969)] was transformed using the reaction solution by the method of Scott, et al. [Katsuya Shigesada: Saibo Kogaku, 2, 616 (1983)] to obtain $Ap^R$colonies. A plasmid DNA psGHIJ1 as illustrated in Fig. 1 was recovered from one of the colonies. The structure of psGHIJ1 was recognized by the cleavage with 5 restriction enzymes (Eco RI, HindIII, ClaI, BglII and StuI) and agarose gel electrophoresis.

Then, 5 μg of the thus constructed plasmid psGHIJ1 was dissolved in 40 μl of Y-100 buffer solution, and 10 units of HindIII was added. Digestion reaction was carried out at 37°C for 3 hours.

In order to ligate the vector DNA and the DNA containing the gene coding for the salmon growth hormone, a DNA linker as set forth below was synthesized.

```
        XhoI                                    HindIII
   5' | TCGAGCAACAAATAGAGCACACAAATCA          3'   +chain
   3'       CGTTGTTTATCTCGTGTGTTTAGTTCGA      5'   -chain
```

Two single chain DNAs of 28-mer were synthesized by a conventional triester method [R. Crea, et al.: Proc. Natl. Acad. Sci., USA, 75, 5765 (1978)]. Twelve pmole each of the single chain DNAs (+chain and -chain) of 28-mer were dissolved in 20 µℓ of a solution consisting of 50 mM Tris-HCl (pH 7.5), 10 mM $MgCl_2$, 10 mM dithiothreitol and 1 mM ATP. Six units of T4 polynucleotide kinase (product of Takara Shuzo Co.) was added and phosphorylation reaction was carried out at 37°C for 60 minutes.

0.1 pmole of the plasmid (about 3.5 Kb) obtained by digesting psGHIJ1 with HindIII was dissolved in 30 µℓ of a solution consisting of 50 mM Tris-HCl (pH 7.5), 10 mM $MgCl_2$, 10 mM dithiothreitol and 1 mM ATP, and 5 µℓ of the synthetic DNA phosphorylation reaction solution mentioned above was added. Six units of T4 ligase (product of Takara Shuzo Co.) was added to the mixture and ligation reaction was carried out at 4°C for 18 hours.

Escherichia coli HB101 was transformed using the reaction solution by the method of Scott, et al. to obtain an $AP^R$ colony. A plasmid DNA psGHIJL2 as illustrated in Fig. 1 was recovered from the colony. The structure of psGHIJL2 was recognized by the cleavage with 6 restriction enzymes (EcoRI, HindIII, XhoI, Cla I, BglII and StuI) and agarose gel electrophoresis.

Then, 5 µg of plasmid psGHIJL2 constructed as mentioned above was dissolved in 40 µℓ of Y-100 buffer solution. Ten units each of XhoI and PstI were added and digestion reaction was carried out at 37°C for 3 hours. After heat treatment at 65°C for 10 minutes, about 0.5 µg of XhoI-PstI DNA fragment (about 2570 bp) containing the gene coding for the salmon growth hormone and Tlpp was obtained by LGT method.

Separately, 5 µg of pAM82 [Proc. Natl. Acad. Sci., USA, 80, 1 - 5 (1983)] was dissolved in 40 µℓ of Y-100 buffer solution. Ten units each of Xho I and PstI were added and digestion reaction was carried out at 37°C for 3 hours. After heat treatment at 65°C for 10 minutes, about 0.7 µg of PstI-XhoI DNA fragment (about 6.8 Kb) containing $P_{PHO5}$ (the promoter of acid phosphatase), leu2 marker, 2 µm origin and a part of arsI gene and about 0.3 µg of PstI-PstI DNA fragment (about 1.4 Kb) containing a part of arsI gene were obtained by LGT method.

0.15 pmole of XhoI-PstI DNA fragment (about 2570 bp) obtained from psGHIJL2 as mentioned above, 0.20 pmole of PstI-PstI DNA fragment (about 1.4 Kb) obtained from pAM82 and 0.10 pmole of PstI-XhoI DNA fragment (about 6.8 Kb) were dissolved in 30 µℓ of T4 DNA ligase buffer solution. Two units of T4 DNA ligase was added and ligation reaction was carried out at 4°C for 18 hours.

Escherichia coli HB101 was transformed using the reaction solution to obtain $Ap^R$ colonies. A plasmid DNA pYSHB5 as illustrated in Fig. 2 was recovered from one of the colonies. The structure of pYSHB5 was recognized by the cleavage with 6 restriction enzymes (EcoRI, HindIII, Bgl II, StuI, XhoI and PstI) and agarose gel electrophoresis.

Example 2

Production of the salmon growth hormone polypeptide by the yeast carrying pYSHB5:

Yeast SHY3 strain was transformed using recombinant plasmid pYSHB5 obtained in Example 1, by the lithium method [H. Ito, et al.: Journal of Bacteriology 153 , 163 (1983)]. The colony which recovered leu2 was obtained and inoculated into 8 mℓ of high phosphate Burkholder minimal medium (2.0% glucose, 0.2% asparagine, 0.15% $KH_2PO_4$, 0.033% $CaCl_2 \cdot 2H_2O$, 0.05% $MgSO_4 \cdot 7H_2O$, 0.01 ppm KI, trace elements, vitamines, 20 µg/mℓ histidine, 20 µg/mℓ tryptophan, 20 µg/mℓ uracil, 20 µg/mℓ adenine, pH 5.0). Culturing was carried out at 30°C for 18 hours. The culture broth was centrifuged at 8,000 rpm for 10 minutes under sterile conditions to recover cells. The cells were suspended in 8 mℓ of phosphate-free Burkholder minimal medium which was prepared by replacing 0.15% $KH_2PO_4$ of the high phosphate minimal medium by 0.15% KCl and cultured at 30°C for 24 hours. The culture broth was centrifuged at 8,000 rpm for 10 minutes to recover cells. The cells were suspended in 0.5 mℓ of 40 mM phosphate buffer solution (pH 7.0) containing 1 mM phenylmethylsulfonylfluoride (PMSF). About 1.5 g of glass beeds (product of TOSHIN RIKO Co.) was added and cells were disrupted under ice cooling for 5 minutes. Then, cells were suspended in Sample

Buffer of Laemmli and subjected to SDS-polyacrylamide gel electrophoresis and Coomassie Brilliant Blue staining to detect a polypeptide band at the portion of a molecular weight of about 25,000 which is the molecular weight of salmon growth hormone. The band was not observed in the case of using yeast SHY3 which does not carry the plasmid.

It was also recognized that the band was reacted with anti salmon growth hormone monoclonal antibody prepared using the salmon growth hormone polypeptide expressed in Escherichia coli. As the results, it was confirmed that yeast SHY3/pYSHB5 carrying pYSHB5 produced salmon growth hormone polypeptide. SHY3/pYSHB5 strain was deposited with Fermentation Research Institute, Agency of Industrial Science and Technology (FRI) on March 24, 1986 as Saccharomyces cerevisiae SHY3/pYSHB5 (FERM BP-1001).

Reference Example 1

Construction of recombinant plasmid psGHIB2 coding for the mature salmon growth hormone:

In this example, 5 µg of plasmid psGHI (JP-A-15699/86) containing a DNA coding for the salmon growth hormone was dissolved in 40 µl of a solution comprising 20 mM Tris-HCl (pH 7.5), 10 mM $MgCl_2$ and 10 mM NaCl (referred to as "Y-10 buffer solution" hereinafter). Then, 10 units of restriction enzyme MboII (product of New England Biolabs Co.) was added and digestion reaction was carried out at 37°C for 3 hours. The concentration of NaCl in the solution was adjusted to 175 mM and 10 units of SalI was added. Digestion reaction was carried out at 37°C for 3 hours. About 0.2 µg of DNA fragment of 163 bp corresponding to N-terminal region was obtained from the reaction solution by LGT method.

Then, 5 µg of psGHI was dissolved in 40 µl of a solution consisting of 20 mM Tris-HCl (pH 7.5), 10 mM $MgCl_2$ and 100 mM NaCl (referred to as "Y-100-2 buffer solution"). Ten units of BamHI was added and digestion reaction was carried out at 37°C for 3 hours. Then, the concentration of NaCl in the reaction solution was adjusted to 175 mM and 10 units of SalI was added. Digestion reaction was carried out at 37°C for 3 hours. About 0.5 µg of a DNA fragment of about 900 bp containing C-terminal region and 3'-non-translational region was obtained from the reaction solution by LGT method.

Separately, 5 µg of pGELI was dissolved in 40 µl of Y-100-2 buffer solution and 10 units each of BamHI and HindIII were added. Digestion reaction was carried out at 37°C for 3 hours. About 1 µg of a DNA fragment of 2.7 Kb containing a tryptophan promoter was obtained from the reaction solution.

In order to add a translational initiation codon ATG necessary for the expression of the DNA coding for the mature salmon growth hormone and to ligate a vector DNA and the DNA mentioned above, a DNA linker as set forth below was synthesized.

```
HindIII                                  MboII
5'- A G C T T A T G A T A G A A A A C   -3'  17 mer
3'-           A T A C T A T C T T T T   -5'  12 mer
                Met   Ile   Glu   Asn
```

Two single chain DNAs of 17-mer and 12-mer were synthesized by a conventional triester method. Then, 12 pmole each of the 17-mer and 12-mer DNAs were dissolved in 20 µl of a solution consisting of 50 mM Tris-HCl (pH 7.5), 10 mM $MgCl_2$, 10 mM dithiothreitol and 1 mM ATP. Six units of T4 polynucleotide kinase (product of Takara Shuzo Co.) was added and phosphorylation reaction was carried out at 37°C for 60 minutes.

Then, 0.1 pmole of MboII-SalI fragment (163 bp) of psGH1, 0.06 pmole of SalI-BamHI fragment (about 900 bp) of psGH1 and 0.02 pmole of HindIII-BamHI fragment (about 2.7 Kb) of pGEL1 obtained above were dissolved in 30 µl of a solution consisting of 50 mM Tris-HCl (pH 7.5), 10 mM $MgCl_2$, 10 mM dithiothreitol and 1 mM ATP. Five µl of the synthetic DNA phosphorylation reaction solution obtained above was added. Six units of T4 DNA ligase (product of Takara Shuzo Co.) was added to the mixture and ligation reaction was carried out at 4°C for 18 hours.

Escherichia coli HB101 was transformed using the reaction solution to obtain $Ap^R$ colonies. A plasmid DNA psGHIB2 as illustrated in Fig. 1 was recovered from one of the colonies. The structure of psGHIB2 was recognized by the cleavage with EcoRI, HindIII, ClaI, BglII, SalI and BamHI and agarose gel electrophoresis. The sequence of the DNA coding for N-terminal region of the salmon growth hormone in psGHIB2 was determined according to the method of Sanger [Sanger, et al.: Proc. Natl. Acad. Sci. USA, 74, 5463 (1977); Amersham Co., M13 cloning and sequencing handbook] using M13 phage and illustrated below.

```
HindIII                                   MboII
A A G C T T A T G A T A G A A A A C C A A
T T C G A A T A C T A T C T T T T G G T T
              Met   Ile   Glu   Asn   Gln
```

As the result, it was confirmed that psGHIB2 contains the DNA coding for the mature salmon growth hormone polypeptide. Escherichia coli containing plasmid psGHIB2 was deposited with the FRI as Escherichia coli ESGHIB2 under FERM BP-612 on September 20, 1984.

## Claims

A process for producing a fish growth hormone polypeptide, which comprises culturing in a medium a yeast containing a recombinant DNA wherein a DNA coding for a fish growth hormone polypeptide is incorporated, accumulating the fish growth hormone polypeptide in the cultured cells and recovering the polypeptide therefrom.

2. The process according to claim 1, wherein the fish growth hormone polypeptide is an Oncorhynchus keta growth hormone having the nucleotide sequence as illustrated in Table 1.

3. The process according to claim 1, wherein the yeast belongs to the genus Saccharomyces.

4. The process according to claim 3, wherein the yeast belongs to the species Saccharomyces cerevisiae.

5. The process according to claim 1, wherein the recombinant DNA is a recombinant DNA having additionally a replication origin and a marker of leu2 originating from yeast.

6. A recombinant DNA, which contains a DNA coding for a fish growth hormone polypeptide, and a replication origin and a marker of leu2 originating from yeast.

7. The recombinant DNA according to claim 6, which is plasmid pYSHB5 having the restriction map as illustrated in Fig. 2.

8. A yeast belonging to the genus Saccharomyces which carries a recombinant DNA wherein a DNA coding for Oncorhynchus keta growth hormone is incorporated.

9. The yeast according to claim 8, which is Saccharomyces cerevisiae SHY3/pYSHB5 (FERM BP-1001).

FIG. 1

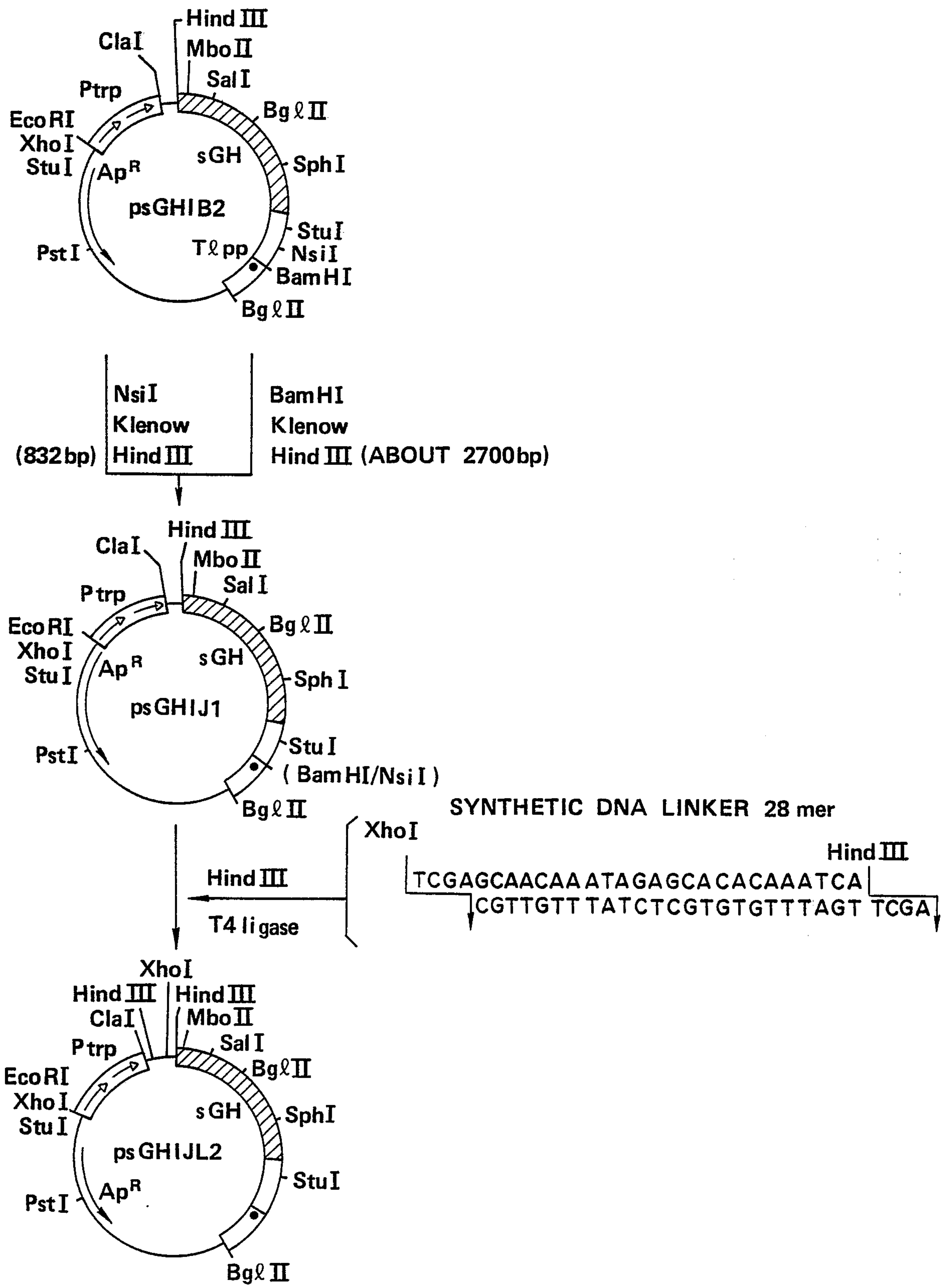
Hind III
Mbo II
Cla I
Sal I
Ptrp
Bgℓ II
EcoRI
Xho I
Stu I
sGH
Sph I
ApR
psGHIB2
Pst I
Tℓpp
Stu I
Nsi I
BamHI
Bgℓ II
NsiI
Klenow
(832bp)
Hind III
BamHI
Klenow
Hind III (ABOUT 2700bp)
psGHIJ1
(BamHI/NsiI)
SYNTHETIC DNA LINKER 28 mer
Xho I
Hind III
TCGAGCAACAAATAGAGCACACAAATCA
CGTTGTTTATCTCGTGTGTTTAGTTCGA
T4 ligase
psGHIJL2

FIG.2

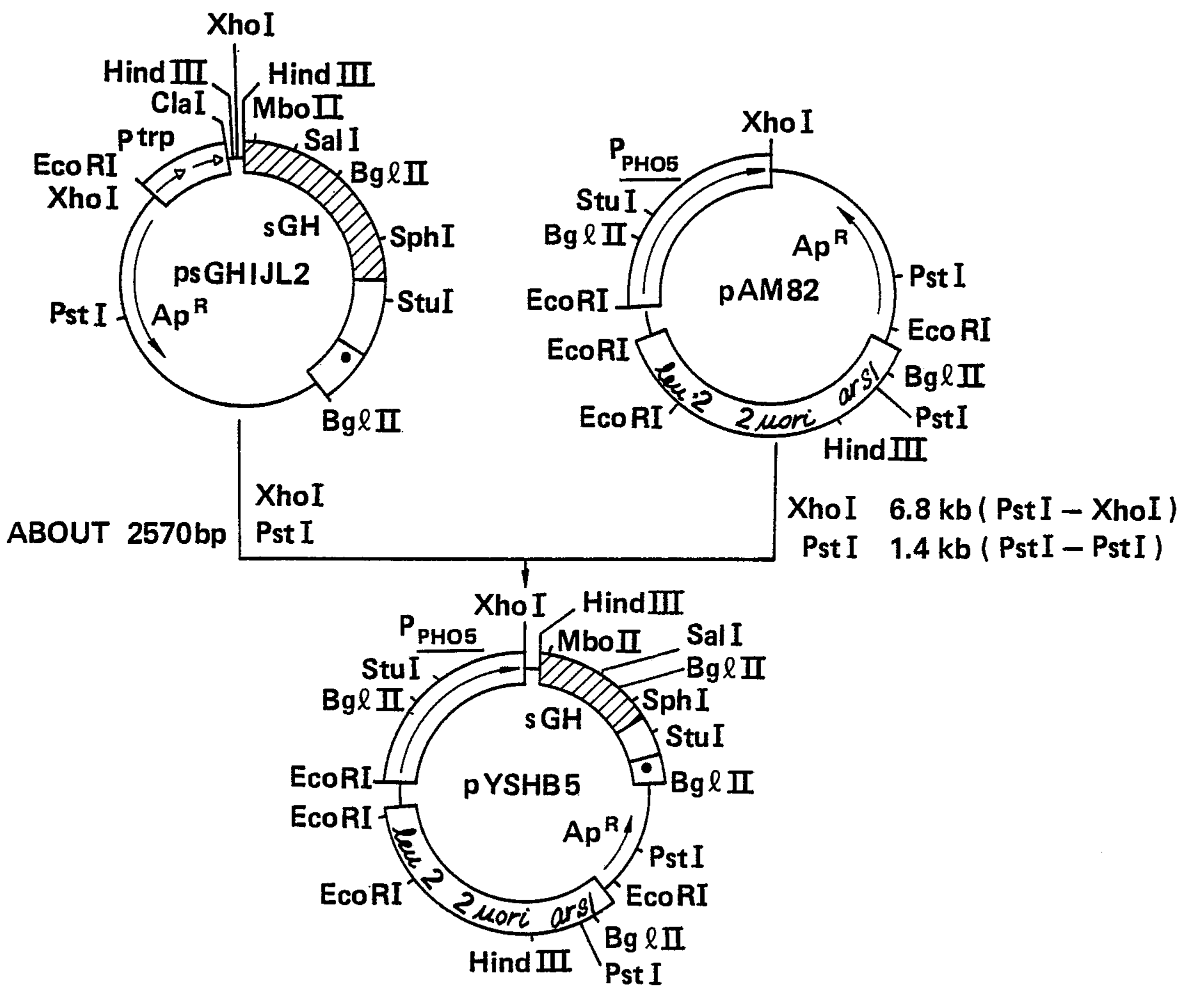
XhoI
HindIII
ClaI
Ptrp
EcoRI
XhoI
HindIII
MboII
SalI
BglII
sGH
SphI
psGHIJL2
StuI
PstI
Ap R
BglII
XhoI
P PHO5
StuI
BglII
Ap R
PstI
EcoRI
pAM82
EcoRI
EcoRI
leu 2
2 μori
ars1
BglII
PstI
EcoRI
HindIII
ABOUT 2570bp
XhoI
PstI
XhoI 6.8 kb ( PstI – XhoI )
PstI 1.4 kb ( PstI – PstI )
XhoI
HindIII
P PHO5
MboII
SalI
StuI
BglII
BglII
SphI
sGH
StuI
EcoRI
pYSHB5
BglII
EcoRI
Ap R
PstI
EcoRI
leu 2
2 μori
ars1
EcoRI
BglII
HindIII
PstI